# EUROPEAN PATENT APPLICATION

(11) **EP 3 456 373 A1**
(43) Date of publication of application: **20.03.2019**
(21) Application number: 17796151.3
(22) Date of filing: 09.05.2017
(51) Int. Cl.: A61M 37/00, B23K 26/352

(54) **LASER MACHINING METHOD, AND MICRONEEDLE MANUFACTURING METHOD**

(30) Priority: 09.05.2016 JP 2016093632
(71) Applicant: Think-Lands Co., Ltd., Yokohama-city, Kanagawa 230-0046 (JP); National University Corporation Chiba University, Chiba-shi, Chiba 267-8522 (JP)
(72) Inventor: MIYAJI Kunio, Yokohama-city Kanagawa 230-0046 (JP); OIKAWA Yoichi, Yokohama-city Kanagawa 230-0046 (JP); OMATSU Takashige, Chiba-city Chiba 263-8522 (JP)
(74) Representative: Plougmann Vingtoft a/s
(86) International application number: PCT/JP2017/017588
(87) International publication number: WO 2017/195790

(57) **Abstract**

[Problem] To make it possible to form micro-projections on a workpiece that contains a macromolecule as a major component, with optical vortex technology.

[Solution] A laser beam machining method of the present invention is to irradiate a workpiece containing a macromolecular compound as a major component with pulsed light of a circularly polarized optical vortex beam which has a wavelength in a range from 1.0 to 1.0 µm and in which the rotation direction of its circular polarization is the same as that of the optical vortex beam, in order to thereby form micro-projections on the workpiece.

## Description

### Technical Field

The present invention is suitable for fabricating microneedles made of bioabsorbable polymers using pulsed light of a circularly polarized optical vortex beam in which the rotation direction of its circular polarization is the same as that of the optical vortex beam, for example.

### Background Art

Over recent years, a great deal of attention has been paid to optical vortex technology using a circularly polarized optical vortex beam in which the rotation direction of its circular polarization is the same as that of the optical vortex beam, in various fields. A circularly polarized optical vortex beam is a laser beam with helical phase wavefronts and is obtained by passing a circularly polarized laser beam through a special spiral phase plate. The circularly polarized optical vortex beam has different properties from typical circularly polarized laser beams.

There has been proposed a technique for forming projections (needle-shaped) on the surface of a workpiece utilizing an ablation phenomenon, in which evaporation occurs from the surface of the workpiece during irradiation of the workpiece with such a circularly polarized optical vortex beam (for example, please see PTL1).

### Citation List

### Patent Literature

PTL1: Japanese Patent No. 5531261

### Summary of Invention

### Technical Problem

However, the above-described optical vortex technology has a problem in that this technology is premised on using semiconductors or metals as workpieces, but not using so-called organic macromolecular compounds, e.g., plastic materials or biopolymers.

The present invention has been made in view of the foregoing problem and intends to provide a laser beam machining method and microneedle fabrication method that are able to form micro-projections on organic macromolecular compounds.

### Solution to Problem

To solve the above problem, the laser beam machining method of the present invention is to irradiate a workpiece containing a macromolecular compound as a major component with pulsed light of a circularly polarized optical vortex beam which has a wavelength in a range from 1.0 to 10.0 µm and in which the rotation direction of its circular polarization is the same as that of the optical vortex beam, in order to thereby form micro-projections on the workpiece.

Further, the microneedle fabrication method of the present invention is to irradiate a workpiece containing a bioabsorbable polymer as a major component with pulsed light of a circularly polarized optical vortex beam which has a wavelength in a range from 1.0 to 10.0 µm and in which the rotation direction of its circular polarization is the same as that of the optical vortex beam, in order to thereby form micro-projections on the workpiece.

### Advantageous Effects of Invention

The present invention provides a laser beam machining method and microneedle fabrication method that are able to form micro-projections on macromolecular compounds.

### Brief Description of Drawings

FIG. 1 is a schematic diagram illustrating how to form a micro-projection.
FIG. 2 is a schematic diagram illustrating an example of an optical system for implementing a laser beam machining method.
FIG. 3 is an enlarged view of a micro-projection formed in a first example.
FIG. 4 is an enlarged view and cross-sectional waveform of a micro-projection formed in a second example.
FIG. 5 is an enlarged view of a micro-projection formed in a third example.

### Description of Embodiments

### (First Embodiment)

Hereinafter, preferred embodiments of the present invention will be described with reference to the accompanying drawings.

Over recent years, microneedles have been proposed, which consist of micro-projections with small diameter, in order to deliver cosmetic ingredients or medical drugs into skin with as little injury to the skin as possible, for cosmetic or medical uses (for example, please see Japanese Patent No. 5495034). These microneedles themselves contain cosmetic ingredients or medical drugs and are absorbed into a body as they are when inserted into skin.

As microneedles have smaller diameter, patients feel less pain and have less injury to their skin. Therefore, there has been a demand that microneedles have smaller diameter and larger aspect ratio. Pattern transfer techniques deal with micro-projections whose base diameter is about 200 µm at the minimum. The use of such pattern transfer techniques increases a risk of breaking needles when removing them from a mold, as the needles have smaller diameter and larger aspect ratio. Thus, it is difficult to fabricate more tiny microneedles using the pattern transfer techniques.

A technique for forming micro-projections on organic macromolecular compounds is considered applicable to various uses, other than the above-described microneedles.

As described earlier, the optical vortex technology is processing technology based on the premise that semiconductors or metals are used as workpieces, and there have been no examples of using macromolecular compounds as workpieces.

The inventors of the present application have made the present invention as a result of making attempts to form micro-projections with so-called optical vortex technology using a circularly polarized optical vortex beam. The inventors have found that the present invention makes it possible to fabricate microneedles that consist of micro-projections with a base diameter of 20 µm or even less.

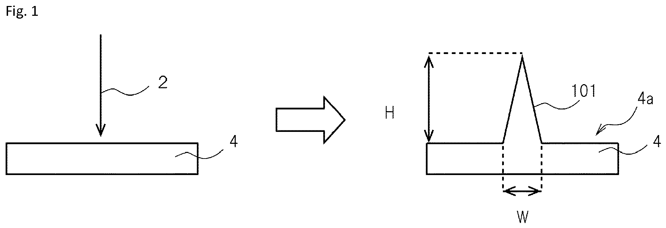

In the invention of the present application, a micro-projection 101 that projects from a workpiece 4 is formed by irradiating the surface 4a of the workpiece 4 with pulsed light 2 of a circularly polarized optical vortex beam, as illustrated in FIG. 1.

A workpiece containing a macromolecular compound as a major component is used. In this specification, the macromolecular compound refers to an organic compound with a weight-average molecular weight Mw of 5000 or more. The major component means that the macromolecular compound accounts for 50 weight% or more of the workpiece. The macromolecular compound preferably has a Tg (glass transition temperature) of 50°C or higher, and more preferably has a Tg of 70°C or higher. This is because, if the Tg is low, the macromolecular compound is difficult to treat at room temperature.

As the macromolecular compound, only one kind of macromolecule or a mixture of two or more kinds of macromolecules may be used. In this connection, the ratio of the major component represents its weight measured after all processing steps are done to the workpiece, and the weight of a solvent component that is evaporated intentionally by a drying step after formation of micro-projections is not included here. That is, the ratio is a ratio obtained after the processing of the workpiece is completed.

Any of known compounds (synthetic polymers and natural polymers) is usable as the macromolecular compound. Examples of such compounds include bioabsorbable polymers, in addition to various kinds of plastic materials including PET (polyethylene terephthalate), polyethylene, polypropylene, acrylic resin, epoxy resin, and polystyrene.

Any of known compounds (synthetic polymers and natural polymers) are usable as the bioabsorbable polymers. Examples of such compounds include ester compounds such as polylactic acid, polyglycolic acid, poly-ε-caprolactone, poly-p-dioxane, and poly(malic acid), acid anhydride such as polyacid anhydride, orthoester compounds such as polyorthoester, carbonate compounds such as polycarbonate, phosphazene compounds such as poly(diaminophosphazene), peptide compounds such as synthetic polypeptide, phosphate ester compounds such as polyphosphoester urethane, carbon-carbon compounds such as polycyanoacrylate, poly-β-hydroxybutyric acid, ester compounds such as poly(malic acid), polyamino acid, chitin, chitosan, hyaluronic acid, sodium hyaluronate, pectic acid, galactan, starch, dextran, dextrin, alginic acid, sodium alginate, cellulose compounds (ethyl cellulose, carboxymethyl cellulose, hydroxyethyl cellulose, hydroxypropyl cellulose, hydroxypropyl methylcellulose, methylcellulose), glycoside compounds (polysaccharides) such as gelatin, agar, Keltrol, Rheozan, xanthan gum, pullulan, and gum arabic, peptide compounds (peptide, protein) such as collagen, gelatin, fibrin, gluten, and serum albumin, phosphate ester compounds (nucleic acids) such as deoxyribonucleic acid and ribonucleic acid, and vinyl compounds such as polyvinyl alcohol.

The circularly polarized optical vortex beam used in the present invention is a circularly polarized laser beam with helicity, and is pulsed light in which the rotation direction of circular polarization is the same as that of an optical vortex beam. The pulse duration of the pulsed light is appropriately selected according to the material of the workpiece 4 or the size of micro-projections to be formed, and is preferably 10 picoseconds or longer and 100 nanoseconds or shorter.

Examples of the circularly polarized optical vortex beam include a Laguerre-Gaussian beam, a Bessel-Gaussian beam, and a multiplexed optical vortex with a plurality of phase singularities in a wavefront. Each of the Laguerre-Gaussian beam and Bessel-Gaussian beam is an eigenmode in the cylindrical coordinates. The Laguerre-Gaussian beam has a diameter with a refractive index profile or gain distribution proportional to the square of the radius vector, whereas the Bessel-Gaussian beam does not have such a diameter.

The Laguerre-Gaussian beam is a typical example of optical vortex beams, and the intensity along the optical axis equals zero (phase singularity) and the cross section along the optical axis has a ring-shaped intensity distribution. Like a spiral staircase, in the Laguerre-Gaussian beam, the phase changes by an integral multiple of 2π when the beam rotates once about the optical axis, and the equiphase surface has a helical shape. This integer indicates a vortex number in the Laguerre-Gaussian beam. In the case where the vortex number is a negative integer, this means a reversed rotation direction.

As with the Laguerre-Gaussian beam, in the Bessel-Gaussian Beam, the phase changes by an integer multiple of 2π when the beam rotates once about the optical axis, like a spiral staircase, and the equiphase surface has a helical shape. This integer indicates a vortex number in the Bessel-Gaussian beam. Examples of the multiplexed optical vortex with a plurality of phase singularities in a wavefront include a double optical vortex and a triple optical vortex. The double optical vortex has two phase singularities and two vortices, and the vortices have vortex numbers of +1 and -1, respectively. The triple optical vortex has three phase singularities and three vortices, and the vortices have vortex numbers of +1, +1, and -1, respectively.

That is, the circularly polarized optical vortex beam is an optical vortex beam in which the spin angular momentum associated with circular polarization is added to the orbital angular momentum corresponding to the vortex number of the optical vortex beam. In the circularly polarized optical vortex beam according to the present invention, the sign of the orbital angular momentum corresponding to the vortex number of the optical vortex beam is the same as the sign of the spin angular momentum associated with the circular polarization. In other words, the rotation direction of the optical vortex is the same as the rotation direction of the circular polarization. This is because, if these signs are opposite, i.e., if their rotation directions are opposite to each other, the orbital angular momentum of the optical vortex and the spin angular momentum of the circular polarization are cancelled out.

In the laser beam machining method and microneedle fabrication method according to the present invention, a method of generating an optical vortex beam is not limited to any specific method. Examples of the method of generating an optical vortex beam include a method of generating an optical vortex beam using a fork-shaped hologram displayed on a liquid crystal spatial modulator, a method of generating an optical vortex beam using a spiral phase plate, a method of generating an optical vortex beam by performing conversion of Hermite-Gaussian mode, and a method of outputting an optical vortex beam directly from a laser resonator.

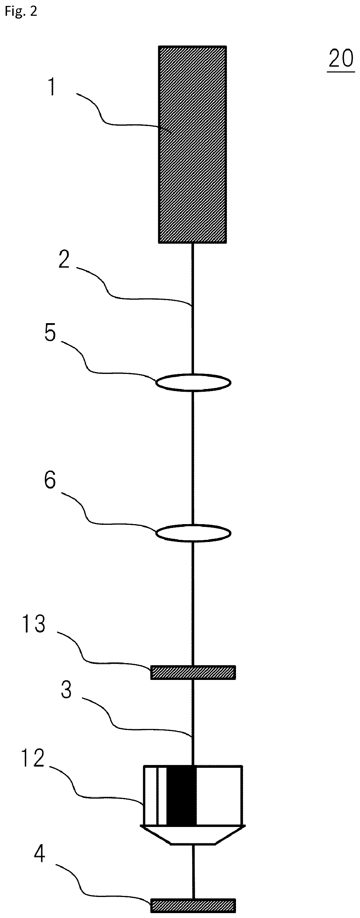

FIG. 2 illustrates an optical system 20 for generating an optical vortex beam using a spiral phase plate.

The laser oscillator 1 is not limited to any specific device, and in this example, a Nd:YAG laser is used as the laser oscillator 1. The laser oscillator 1 oscillates linearly polarized pulsed light 2 with Q-switching. The pulse duration of the linearly polarized pulsed light 2 is 10 picoseconds or longer and 100 nanoseconds or shorter. This is because, if the pulse duration is shorter than 10 picoseconds, plasma is less likely to be generated, and if the pulse duration exceeds 100 nanoseconds, HAZ problems occur. In the case where the pulse duration is 10 picoseconds or longer, the light and a workpiece interact with each other sufficiently.

A wavelength of 1.0 µm or longer and 10.0 µm or longer is used as the wavelength of the linearly polarized pulsed light 2 that is oscillated by the laser oscillator 1. Unlike inorganic substances, macromolecular compounds go through a carbonization phenomenon, in which the compounds are decomposed and carbonized by heating. Therefore, if laser light with high energy and short wavelength is used, ablation (sublimation) and carbonization are likely to occur in a macromolecular compound at the same time, and it is very difficult to control the laser light. On the other hand, laser light with a wavelength of 10.0 µm or longer has too low energy, which decreases the efficiency and enlarges the minimum diameter at the time of light convergence. Therefore, it is difficult to control the spot size. Because of the same reasons as above, the pulsed light 2 preferably has a wavelength longer than or equal to 1.2 µm and shorter than 7.0 µm.

More specifically, the pulsed light 2 preferably has a wavelength longer than or equal to 1.5 µm and shorter than 4.0 µm. A near-infrared region of 1.5 µm to 2.0 µm is preferable because absorption of combination tone of hydrogen bonds such as C-H, O-H, and N-H is observed, its absorbance is relatively small, and the energy of wavelength and the absorption amount are balanced. In addition, in an infrared region of 2.0 µm to 4.0 µm, absorption specific to organic macromolecular compounds is often observed, its absorbance is very high, and it is possible to cause ablation even with low wavelength energy. Especially, a region of 2.0 µm to 3.7 µm is preferable because a broad absorption band of hydroxyl group and amide group exists and a slight wavelength deviation is absorbed. In this connection, for example, the wavelength of the pulsed light 2 may be adjusted, by an optical parametric oscillation (OPO) using a KTP crystal (KTiOPO₄) or by upconversion of CO₂ laser radiation.

In the laser beam machining method of the present invention, the output of a laser oscillator may be set to have a preset peak power density, and is appropriately selected according to various factors including the spot diameter of pulsed light 3 of an optical vortex beam on the workpiece 4, the material of the workpiece 4, and the wavelength of pulsed light 2. Although the output of the laser oscillator is not particularly limited, it is preferably in a range from 0.1 mJ to 10 mJ. This is because, if the output is too small, ablation does not occur or is insufficient, and if the output is too large, the workpiece 4 is carbonized. The spot diameter of the pulsed light 3 of the optical vortex beam on the workpiece 4 is appropriately determined according to the size of micro-projections to be formed, and is preferably 10 µm or more and 300 µm or less, but is not particularly limited.

The linearly polarized pulsed light 2 oscillated by the laser oscillator 1 passes through a lens 5 with a focal length of 50 mm and a lens 6 with a focal length of 300 mm, so that the beam size is increased by six-fold. After that, the pulsed light 2 passes through a 12-divided spiral phase plate 13, so that the pulsed light 2 is converted into the pulsed light 3 of the optical vortex beam. The lens 5 with a focal length of 50 mm and the lens 6 with a focal length of 300 mm are spaced 350 mm apart. This is to improve the quality of the beam by effectively using the area of the spiral phase plate 13 and to avoid damaging the spiral phase plate 13. The focal lengths are not particularly limited. After that, the pulsed light 3 is focused by an objective lens 12 (with a focal length of 50 mm) onto the workpiece 4. The magnification of the objective lens 12 is determined according to a desired spot diameter, but is not particularly limited. In this example, the magnification of the objective lens 12 is in a range from 5 to 50. The focal length of the objective lens 12 is not particularly limited.

The spiral phase plate 13 is a phase plate having a thickness distribution adjusted so as to provide a passing laser beam with a prescribed phase distribution. The thickness distribution of the phase plate is approximated by a stepped discontinuous distribution, and the number of steps corresponds to the number of divisions. The number of divisions for the spiral phase plate 13 is not particularly limited, and for example, the spiral phase plate 13 divided into 12 or 16 parts is used. Note that an optical vortex beam may be generated using a fork-shaped hologram displayed on a liquid crystal spatial modulator, instead of using the spiral phase plate 13. This type of optical system is described in PTL1. In addition, any optical vortex oscillation apparatus described in PTL3 to PTL5 may be used.

PTL3: Japanese Patent No. 5831896
PTL4: Japanese Patent Application No. 2013-519522
PTL5: Japanese Patent No. 5035803

In the laser beam machining method of the present invention, the optical vortex beam is a Laguerre-Gaussian beam or a Bessel-Gaussian beam. A vortex number is preferably an integer of 1 or greater or -1 or less, and more preferably an integer of 2 or greater or -2 or less. This is because, with an increase in the absolute value of the vortex number of the Laguerre-Gaussian beam, a processed surface gets more smoothed. A method of generating a Laguerre-Gaussian beam having a high vortex number may be implemented by aligning spiral phase plates. For example, the vortex number may be set to two by aligning two spiral phase plates each used for generating a primary vortex. In the method of generating an optical vortex beam using a fork-shaped hologram displayed on a liquid crystal spatial modulator, the vortex number may be set to two by using a three-fork-shaped hologram displayed on a phase plate liquid crystal spatial modulator. In addition, in the laser beam machining method of the present invention, the optical vortex beam is preferably a multiplexed optical vortex with a plurality of phase singularities in a wavefront.

### Example(s)

### (First Example)

Micro-projections were formed on a workpiece 4 using an optical system illustrated in FIG. 2.

A laser oscillator 1 was a Nd:YAG laser and had output energy of 0.05 mJ. Pulsed light 2 had a pulse duration of 20 ns and a wavelength of 2480 nm. An objective lens 12 had a focal length of 50 mm and 20-times magnification. The number of divisions for a spiral phase plate 13 was 16. The workpiece 4 was a sheet of sodium hyaluronate (100%). Pulsed light 3 of a generated optical vortex beam was pulsed light of a Laguerre-Gaussian beam, and the vortex number thereof was one. The spot diameter of the pulsed light 3 of the optical vortex beam (pulsed light of a Laguerre-Gaussian beam) on the workpiece 4 was 50 µm.

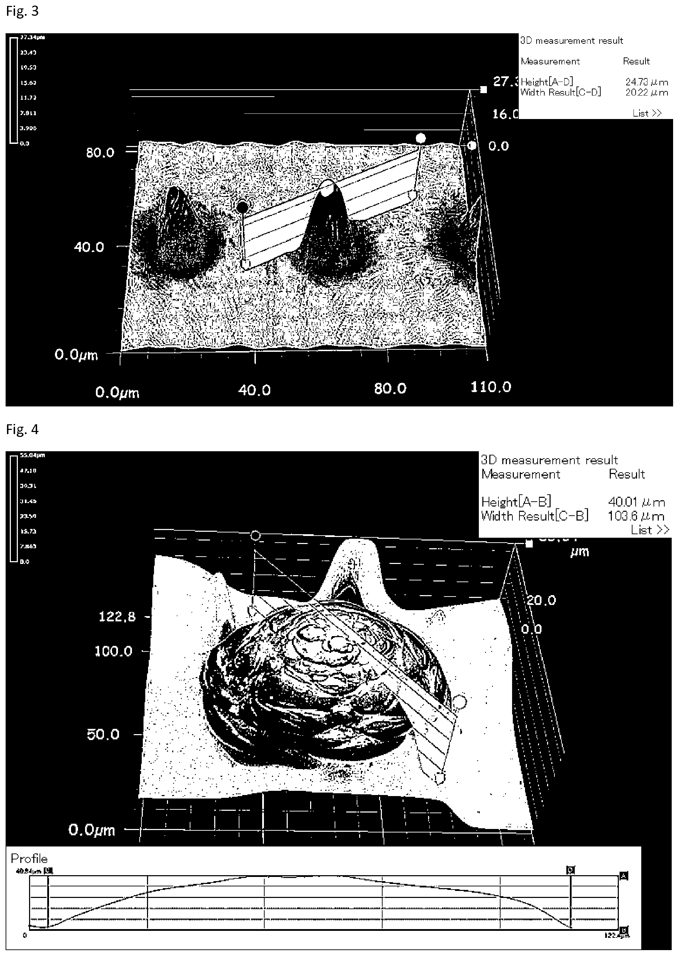

As a result, micro-projections with a base diameter W of approximately 20 µm and a height H of approximately 25 µm were formed as illustrated in FIG. 3. The aspect ratio (height H/diameter W) was approximately 1.25. In this connection, the base diameter is a diameter measured on the baseline (i.e., surface 4a) of the workpiece 4, and the height is a height measured from the baseline.

### (Second Embodiment)

The following describes a second embodiment. The same elements as in the first embodiment will not be described again.

In the present invention, a processing agent that is removable by drying is contained in a workpiece 4. A compound with a weight-average molecular weight Mw of less than 300 is preferably used as the processing agent. Water or organic solvent, which is easily removable by heating, or a compound with absorption properties in the wavelength of an optical vortex beam is particularly preferred. Especially, in the case where microneedles for injection are fabricated, water or ethyl alcohol, which is not harmful to human bodies, is preferably used. The content of the processing agent is not particularly limited, but the content of the processing agent relative to the total amount (weight conversion) in the workpiece 4 is preferably in a range from 95.0% to 2.0%, and more preferably in a range from 70.0% to 5.0%.

For example, in the case where a macromolecular compound is previously dispersed in a processing agent and a drying process is performed to produce the workpiece 4, the workpiece 4 is produced such that a certain amount of processing agent remains under appropriately set drying conditions. Alternatively, after a workpiece 4 is produced, the workpiece 4 may be placed for a certain time under the conditions of high humidity of the processing agent for absorption or may be impregnated into the processing agent, such that the workpiece 4 contains the processing agent.

This allows the processing agent to get into the macromolecular compound. That is to say, the macromolecular compound is in so-called dissolved state. As a result, ablation is easy to occur and thus it is easy to form micro-projections, compared with workpieces containing no processing agent.

After the micro-projections are formed, the workpiece 4 may be dried using a drier. Although there are no restrictions on the drying conditions, the workpiece 4 is dried at a temperature of 50 to 80°C for about one to five hours, for example. In this connection, in the case where a compound (water, ethyl alcohol, or the like) that is not harmful to human bodies is used as the processing agent, the product containing the processing agent may be used as a finished product. The product may be usable as it is without being dried.

### (Second Example)

A sheet of sodium hyaluronate (100%) containing water was used as a workpiece 4. The water content was about 30% of the workpiece 4. In forming the sodium hyaluronate dispersed in water into a sheet shape, the time for drying was shortened so as to allow water to remain in the workpiece 4.

A laser oscillator 1 irradiated the workpiece 4 with pulsed light 3 of an optical vortex beam under the same conditions except that a wavelength of 1064 nm was used.

As a result, a micro-projection with a height H of 40 µm and a base diameter W of 110 µm was formed, as illustrated in FIG. 4.

### (Third Example)

A workpiece produced by causing sodium hyaluronate (100%) to contain a lot of water and then drying the resultant at a prescribed temperature (for example, 40 to 60°C) was used as a workpiece 4. The water content was about 50% of the workpiece 4. In forming the sodium hyaluronate dispersed in water into a sheet shape, the drying temperature and moisture level were adjusted such that the workpiece 4 contains a prescribed amount of water.

A laser oscillator 1 irradiated the workpiece 4 with pulsed light 3 of an optical vortex beam under the same conditions except that a wavelength of 1064 nm was used.

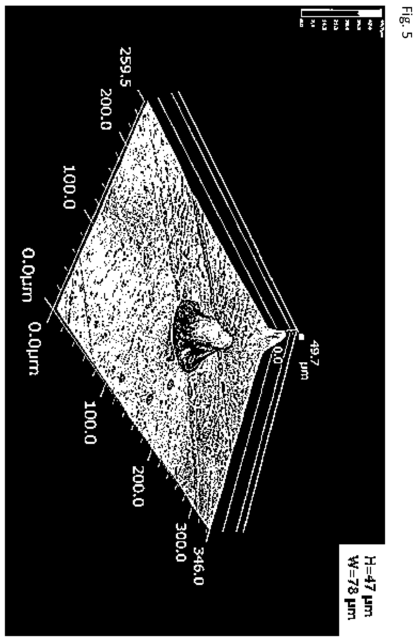

As a result, a micro-projection with a height H of 47 µm and a base diameter W of 78 µm was formed, as illustrated in FIG. 5.

As described above, it was confirmed that an increase in the content of water in a sheet of sodium hyaluronate resulted in an increase in the aspect ratio, which is a ratio of height to base diameter. It is considered that this was resulted from water used as a processing agent. As in the above, by using collagen or the like, which is a water-holding polymer with high water retention capability (30% or more water, more preferably, 100% or more water in relation to a macromolecular compound body), containing 30% or more water by weight, it is possible to form microneedles with a high aspect ratio.

### (Operations and Effects)

With the above configuration, the laser beam machining method of the present invention is to irradiate a workpiece containing a macromolecular compound as a major component with pulsed light of a circularly polarized optical vortex beam which has a wavelength in a range from 1.0 to 10.0 µm and in which the rotation direction of its circular polarization is the same as that of the optical vortex beam, in order to thereby form micro-projections on the workpiece.

The above approach makes it possible to cause ablation without carbonizing the macromolecular compound, which is a major component of the workpiece, and to form micro-projections on the workpiece.

The optical vortex beam has a wavelength in a range from 1.2 to 7.0 µm, so that it is possible to cause ablation with appropriate energy and the wavelength at which the workpiece exhibits the absorption properties.

The optical vortex beam has a wavelength in a range from 1.5 to 4.0 µm, so that it is possible to effectively cause ablation in the workpiece by relatively large absorption.

The workpiece contains a processing agent that is a solvent that is easily removable by heating, so that it is possible to reduce entanglement of macromolecular components and to promote the ablation in the processing agent.

The processing agent is a solvent that is easily removable by heating, so that it is possible to prevent carbonization of the macromolecular compound of the workpiece due to energy absorption by ablation in the solvent or energy absorption under gas phase.

The processing agent exhibits the absorption properties in the wavelength of the optical vortex beam to be emitted, so that it is possible to promote generation of heat in the workpiece and to thereby cause ablation effectively.

The processing agent is water or ethyl alcohol, so that not only absorption of an optical vortex beam and reduction in entanglement of macromolecular compounds occur but also the processing agent is not harmful to human bodies or environment.

The workpiece contains water, an organic solvent, or a mixture thereof as the processing agent, and the processing agent is removed by drying after irradiation with the pulsed light. Thereby, it is possible to produce the workpiece that has the same composition as the original workpiece. It is also possible to reduce the size of micro-projections once formed, utilizing the contraction behavior caused by drying.

The workpiece contains at least water as the processing agent, and the optical vortex beam has a wavelength in a range from 1.7 to 3.5 µm, so that it is possible to cause ablation effectively utilizing high absorption of the optical vortex beam by the OH group of water.

The processing agent contains water or ethyl alcohol as a major component and the processing agent accounts for 20% to 70% by weight of the workpiece at the time of irradiation with the optical vortex beam. In this connection, the major component means that the major component accounts for 50% or more by weight of the processing agent.

Thereby, utilizing the absorption properties of the hydroxyl group in water and ethyl alcohol, it is possible to cause ablation effectively by the use of the optical vortex beam. For example, according to the second and third examples, a lot of water is contained as the processing agent, so that it is possible to increase the efficiency of ablation using the optical vortex beam of 1064 nm that sodium hyaluronate itself does not absorb.

The optical vortex beam has a wavelength in a range from 1.0 to 3.5 µm. Thereby, it is possible to fabricate microneedles using a wavelength region where energy is relatively high.

With the above-described configuration, the microneedle fabrication method of the present invention is to irradiate a workpiece containing a bioabsorbable polymer as a major component with pulsed light of a circularly polarized optical vortex beam which has a wavelength in a range from 1.0 to 10.0 µm and in which the rotation direction of its circular polarization is the same as that of the optical vortex beam, in order to thereby form micro-projections on the workpiece.

The above approach makes it possible to cause ablation without carbonizing the bioabsorbable polymer, which is a major component of the workpiece, and to form micro-projections on the workpiece. As a result, it is possible to fabricate microneedles containing the bioabsorbable polymer as a major component.

### (Other Embodiments)

In the above embodiments, micro-projections with no hollows are formed. Alternatively, micro-projections with hollows at the center may be formed, like injection needles. A hollow may be formed at the center of each micro-projection at the beginning or later.

In addition, in the above embodiments, the present invention is applied for fabricating microneedles for medical and cosmetic uses. Alternatively, for example, micro-projections may be formed on the surface of a polymeric material, such as a plastic material, for industrial uses. In addition, the surface of a workpiece does not need to be flat, and for example, micro-projections may be formed on a curved surface, like the surface of a ball.

### Industrial Applicability

The present invention is applicable to microneedles that are to be attached to human bodies, for example.

### Reference Signs List

1: Laser oscillator
2: Pulsed light
3: Pulsed light
4: Workpiece
4a: Surface
12: Objective lens
13: Spiral phase plate
20: Optical system
101: Micro-projection

## Claims

1. A microneedle fabrication method, comprising:
irradiating a workpiece containing a macromolecular compound as a major component with pulsed light of a circularly polarized optical vortex beam which has a wavelength in a range from 1.0 to 10.0 µm and in which a rotation direction of circular polarization matches a rotation direction of the optical vortex beam, in order to form a micro-projection on the workpiece.

2. The microneedle fabrication method according to claim 1, wherein the optical vortex beam has a wavelength in a range from 1.0 to 7.0 µm.

3. The microneedle fabrication method according to claim 1, wherein the optical vortex beam has a wavelength in a range from 1.5 to 4.0 µm.

4. The microneedle fabrication method according to claim 1, wherein the workpiece contains a processing agent.

5. The microneedle fabrication method according to claim 4, wherein the processing agent is a solvent that is easily removable by heating.

6. The microneedle fabrication method according to claim 4, wherein:
the processing agent is water or ethyl alcohol; and
the processing agent accounts for 20% to 70% by weight of the workpiece when the workpiece is irradiated with the optical vortex beam.

7. The microneedle fabrication method according to claim 6, wherein the optical vortex beam has a wavelength in a range from 1.0 to 3.5 µm.

8. The microneedle fabrication method according to claim 1, wherein:
the workpiece contains water, an organic solvent, or a mixed solvent thereof as the processing agent; and
the processing agent is removed by drying after irradiation with the pulsed light.

9. The microneedle fabrication method according to claim 4, wherein:
the processing agent contains at least water; and
the optical vortex beam has a wavelength in a range from 1.7 to 3.5 µm.

10. A laser beam machining method, comprising:
irradiating a workpiece containing a bioabsorbable polymer as a major component with pulsed light of a circularly polarized optical vortex beam which has a wavelength in a range from 1.0 to 10.0 µm and in which a rotation direction of circular polarization matches a rotation direction of the optical vortex beam, in order to form a micro-projection on the workpiece.
